# NOUVEAU FASCICULE DE BREVET EUROPEEN

(11) **EP 1 908 450 B2**
(45) Date de publication et mention de la décision concernant l'opposition: **14.06.2017**
(45) Mention de la délivrance du brevet: 06.07.2011
(21) Numéro de dépôt: 07115223.5
(22) Date de dépôt: 29.08.2007
(51) Int. Cl.: A61K 8/02, A61K 8/44, A61K 8/60, A61K 8/73, A61K 8/81, A61Q 19/10

(54) **Composition de nettoyage contenant des polymères superabsorbants**
Reinigungszusammensetzung, die superabsorbierende Polymere enthält
Cleaning composition containing superabsorbent polymers

(30) Priorité: 04.10.2006 FR 0654074
(43) Date de publication de la demande: 09.04.2008
(73) Titulaire: L'Oréal, 75008 Paris (FR)
(72) Inventeur: BERNARD, Anne-Laure, 92200, Neuilly / Seine (FR); AUBRUN-SONNEVILLE, Odile, 92160, ANTONY (FR); LEROY, Laurence, 78410, ST REMY LES CHEVREUSES (FR)
(74) Mandataire: Duvert, Sandra

(56) Documents cités:
- EP-A1- 1 800 661
- EP-A2- 1 195 157
- WO-A1-96/07395
- WO-A1-97/45510
- WO-A1-02/056855
- DE-A1- 3 503 458
- FR-A1- 2 805 461
- US-A- 5 703 026
- US-A1- 2003 035 783

## Description

L'invention a pour objet une composition de nettoyage comprenant un polymère superabsorbant convenablement sélectionné, ainsi qu'à ses utilisations dans les domaines cosmétique ou dermatologique, notamment comme produit de nettoyage ou de démaquillage de la peau, des cheveux y compris du cuir chevelu et/ou des muqueuses (lèvres).

Le nettoyage de la peau est très important pour le soin du visage, et il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Les produits de nettoyage moussants actuellement commercialisés sont sous forme de pains, de gels ou de crèmes moussantes, et ils contiennent ou non des savons. Certains consommateurs reprochent aux produits moussants contenant des savons de provoquer des tiraillements dus à leur détergence trop importante. Il est donc souvent préférable de préparer des produits moussants sans savons pour avoir une meilleure tolérance. Les produits de nettoyage ne contenant pas de savons actuellement mis sur le marché sont généralement de faible viscosité et de ce fait, pas toujours pratiques d'utilisation car ils ont tendance à couler.

Afin d'épaissir les produits moussants sans savon, il est connu d'y ajouter des épaississants tels que des composés oxyéthylénés, des polymères comme les gommes de cellulose ou leurs dérivés, les gommes de guar ou leurs dérivés, les polymères acrylique y compris ceux comportant éventuellement une partie hydrophobe et ayant donc un caractère amphiphile. Ainsi, le document EP-A-1,172,095 décrit une composition moussante contenant silice et composé oxyéthyléné tels que par exemple le PEG-120 methyl glucose dioleate.

Toutefois, lorsqu'on augmente le pourcentage de ces composés épaississants pour avoir une composition plus épaisse, cela engendre des inconvénients. Ainsi, lorsqu'on augmente le pourcentage des composés oxyéthylénés, l'étalement sur la peau se fait par paquets et n'est pas homogène. Lorsqu'on augmente le pourcentage en polymère acrylique, les produits obtenus peuvent être visqueux mais conduisent à un démarrage en mousse médiocre. Par ailleurs, l'addition d'un trop grand pourcentage de gomme entraîne un frein dans le démarrage de la mousse et affecte les qualités de la mousse.

Il est donc difficile d'épaissir des milieux moussants tout en conservant les propriétés requises, à savoir un bon mélange à l'eau et une transformation rapide en mousse, et un étalement homogène lors de l'application sur la peau.

Il subsiste donc le besoin de réaliser des compositions moussantes épaisses, présentant une très bonne qualité de mousse et d'étalement.

La demanderesse a découvert de manière surprenante qu'on pouvait atteindre le but de l'invention et obtenir un produit moussant ayant de bonnes propriétés cosmétiques (qualités de la mousse, qualité de l'étalement sur la peau) tout en étant suffisamment épais, en utilisant un polymère superabsorbant se présentant sous forme de particules, dans une composition contenant des tensioactifs moussants non ioniques et/ou amphotères. Ces polymères se présentent sous forme de particules qui gonflent fortement dans l'eau et génèrent de la viscosité par empilement compact, même en milieu tensioactif. Lors de l'application, les tensioactifs sont facilement libérés sous l'effet du cisaillement et ils génèrent de la mousse.

Certes, le document US-5,703,026 décrit des compositions nettoyantes contenant un polymère superabsorbant, mais ces compositions sont des solides (pains ou « bars » en Anglais), et non des gels qui sont des compositions souples. Ainsi, les compositions décrites dans ce document ont une structure tout à fait différente de celle des compositions de la présente invention qui contiennent beaucoup d'eau et doivent avoir de bonnes propriétés moussantes tout en ayant une viscosité satisfaisante pour donner un gel.

Ainsi, la présente demande a pour objet une composition de nettoyage pour application topique, se présentant sous forme d'un gel et contenant, dans un milieu aqueux physiologiquement acceptable, au moins 40% en poids d'eau par rapport au poids total de la composition, au moins un tensioactif moussant choisi parmi les tensioactifs non ioniques et les tensioactifs amphotères, et au moins un polymère superabsorbant se représentant sous forme de particules qui, une fois hydratées, gonflent en formant des billes molles ayant un diamètre moyen en nombre de 10 microns à 1000 microns, la composition étant exempte de tensioactif anionique.

Le polymère superabsorbant se présente sous forme de particules.

On entend ici par « application topique » une application externe sur les matières kératiniques, que sont notamment la peau, le cuir chevelu, les cils, les sourcils, les ongles, les cheveux et/ou les muqueuses. La composition étant destinée à une application topique, elle comprend un milieu physiologiquement acceptable. On entend par « milieu physiologiquement acceptable », un milieu compatible avec la peau, les lèvres, le cuir chevelu, les cils, les yeux, les ongles et/ou les cheveux. La composition peut constituer notamment une composition cosmétique ou dermatologique.

On entend par « gel » une composition gélifiée, qui est souple par opposition à un produit solide, et dont on peut mesurer la viscosité.

Par ailleurs, on entend par « milieu aqueux », un milieu comportant une quantité d'eau d'au moins 40 % en poids, mieux d'au moins 50 % en poids, et encore mieux d'au moins 60 % en poids par rapport au poids total de la composition. Cette quantité peut aller par exemple de 40 à 98 % en poids, mieux de 50 à 80 % en poids et encore mieux de 60 à 80 % en poids par rapport au poids total de la composition. Le milieu aqueux des compositions moussantes de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

On entend par « tensioactif moussant » un tensioactif ayant des propriétés moussantes quand il est introduit dans l'eau. Les tensioactifs moussants sont des détergents et se différencient des tensioactifs émulsionnants par la valeur de leur HLB (Hydrophilic Lipophilic balance), qui est généralement supérieure à 15 et mieux supérieur à 18 ou même à 20, le HLB étant le rapport entre la partie hydrophile et la partie lipophile dans la molécule. Le terme HLB est bien connu de l'homme du métier et est décrit par exemple dans "The HLB system. A time-saving guide to Emulsifier Selection" (publié par ICI Americas Inc ; 1984).

Les compositions de l'invention sont des compositions de nettoyage moussantes, utilisables dans le domaine du nettoyage de la peau, des cheveux ou des muqueuses. Elles sont rincées.

La composition obtenue se présente sous forme d'un gel, et la viscosité des compositions selon l'invention va de préférence de 0,1 à 50 Pa.s, mesurée à 25°C à l'aide du Rheomat RM180 de Rheometric Scientific à 200 RPM (tours par minute), 10 minutes après la mise en rotation du mobile. L'appareil est équipé d'un mobile différent selon les viscosités, par exemple d'un mobile 2 pour les gammes de viscosités inférieures à 0,7 Pa.s, d'un mobile 3 pour les gammes de viscosités de 0,2 à 4 Pa.s, et d'un mobile 4 pour les gammes de viscosités supérieures à 2 Pa.s.

Les polymères superabsorbants permettent d'obtenir des compositions ayant de bonnes propriétés cosmétiques. En outre, ils permettent d'obtenir des textures ayant un aspect givré, c'est-à-dire l'aspect du verre dépoli, qui sont très originales et produisent un effet de scrub doux fondant lors de l'application.

### Polymères superabsorbants

On entend par « polymère superabsorbant », un polymère qui gonfle dans l'eau, notamment un polymère réticulé. Le polymère superabsorbant utilisé dans la composition de l'invention se présente sous forme de particules, qui, une fois hydratées, gonflent en formant des billes molles ayant un diamètre moyen en nombre de 10 µm à 1000 µm. Pour atteindre le but de l'invention et donner un bon épaississement des compositions, les polymères superabsorbants doivent être sous forme de particules.

Les polymères superabsorbants peuvent être en particulier choisis parmi :
- les polyacrylates de sodium réticulés comme par exemple ceux commercialisés sous les dénominations Octacare X100, X110 et RM100 par la société Avecia, ceux commercialisés sous les dénominations Flocare GB300 et le Flosorb 500 par la société SNF, ceux commercialisés sous les dénominations Luquasorb 1280 et Luquasorb 1110 par la société BASF, ceux commercialisés sous les dénominations Water Lock G400 et G430 (nom INCI : Acrylamide/Sodium acrylate copolymer) par la société Grain Processing,
- les amidons greffés par un polymère acrylique (homopolymère ou copolymère) et notamment par le polyacrylate de sodium, tels que ceux commercialisés sous les dénominations Sanfresh ST-100C, ST100MC, IM-300MC par la société Sanyo Chemical Industries (nom INCI Sodium polyacrylate Starch),
- les amidons hydrolysés greffés par un polymère acrylique (homopolymère ou copolymère) et notamment le copolymère acryloacrylamide/acrylate de sodium, comme ceux commercialisées sous les dénominations Water Lock A-240, A-180, B-204, D-223, A-100, C-200, D-223, par la société Grain Processing (nom INCI : Starch/acrylamide/sodium acrylate copolymer),
- les polymères à base d'amidon, de gomme et de dérivé cellulosique, tels que celui contenant de l'amidon, de la gomme de guar et de la carboxymethyl cellulose de sodium, commercialisé sous la dénomination Lysorb 220 par la société Lysac,
- et leurs mélanges.

La quantité de polymère(s) superabsorbant(s) dans la composition de l'invention dépend des tensioactifs présents et de la viscosité souhaitée pour le produit à obtenir. Elle peut aller par exemple de 0,1 à 20 % en poids, de préférence de 0,5 à 20 % en poids, mieux de 0,5 à 10 % en poids, et encore mieux de 0,5 à 5 % en poids par rapport au poids total de la composition.

### Tensioactifs moussants

La composition contient au moins un tensioactif moussant choisi parmi les tensioactifs non ioniques et les tensioactifs amphotères, c'est-à-dire qu'elle peut contenir uniquement un ou plusieurs tensioactifs non ioniques, ou uniquement un ou plusieurs tensioactifs amphotères, ou un mélange d'un ou plusieurs tensioactifs non ioniques et d'un ou plusieurs tensioactifs amphotères.

La composition est exempte de tensioactifs anioniques.

De plus, la composition peut éventuellement contenir un ou plusieurs tensioactifs cationiques tels que par exemple le bromure de myristyl triméthylammonium, mais tensioactifs cationiques doivent être présents en une quantité d'au plus 1 % en poids par rapport au poids total de la composition.

La quantité totale de tensioactifs moussants (en matière active) peut varier selon l'utilisation finale des compositions. Elle peut. aller par exemple de 3 à 20 % en poids et de préférence de 3 à 15 % en poids par rapport au poids total de la composition. On entend par « quantité totale », la quantité de tous les tensioactifs moussants y compris les anioniques et cationiques s'il y en a.

Selon un mode préféré de réalisation, la composition contient au moins un tensioactif non ionique et au moins un tensioactif amphotère. Et selon un mode encore préféré de réalisation, la quantité de tensioactif non ionique est supérieure à la quantité de tensioactif amphotère.

### Tensioactifs non ioniques

On peut utiliser par exemple comme tensioactifs non ioniques, les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10 ® par la société Kao Chemicals, le produit commercialisé sous la dénomination PLANTAREN 2000 UP ® par la société Cognis, et le produit commercialisé sous la dénomination ORAMIX NS 10 ® par la société Seppic ; le caprylyl/capryl glucoside comme le produit commercialisé sous la dénomination ORAMIX CG 110 ® par la société Seppic ; le laurylglucoside comme les produits commercialisés sous les dénominations PLANTAREN 1200 N ® et PLANTACARE 1200 ® par la société Cognis ; et le coco-glucoside comme le produit commercialisé sous la dénomination PLANTACARE 818/UP ® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF@ par la société Chimex.

Selon un mode préféré de réalisation de l'invention, les tensioactifs non ioniques sont choisis parmi les alkyl polyglucosides.

### Tensioactifs amphotères

Les tensioactifs amphotères (ou zwitterioniques) peuvent être choisis par exemple parmi les dérivés de bétaïne, les alkylamphoacétates, les hydroxylsultaines, et leurs mélanges.

Comme dérivés de bétaïnes, on peut citer par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica, la cocamidopropyl betaine commercialisé par exemple sous la dénomination VELVETEX BK 35® par Cognis ou encore le undecylenamidopropyl betaïne commercialisé par exemple sous la dénomination AMPHORAM U® par Ceca.

Comme alkylamphoacétates, on peut citer par exemple le N-cocoyl-N-carboxyméthoxyéthyl-N-carboxyméthyl-éthylènediamine N-di-sodique (nom INCI : disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom INCI: sodium cocamphoacetate).

Comme hydroxylsultaïnes, on peut citer la Cocamidopropyl hydroxysultaïne comme le produit commercialisé sous la dénomination REWOTERIC AM CAS par la société Golschmidt-Degussa.

Selon un mode préféré de réalisation de l'invention, les tensioactifs amphotères sont choisis parmi les dérivés de bétaïne.

Selon un mode plus préféra de réalisation de l'invention, la composition contient au moins un tensioactif non ionique et au moins un tensioactif amphotère.

### Autres tensioactifs

Comme indiqué ci-dessus, la composition de l'invention peut éventuellement contenir un ou plusieurs tensioactifs, cationiques.

Quand la composition contient un ou plusieurs tensioactifs cationiques, la quantité de ces tensioactifs cationiques (en matière active) doit être d'au plus 1 % en poids, mieux d'au plus 0,5 % en poids par rapport au poids total de la composition. Cette quantité peut aller de 0 à 1 % du poids total de la composition.

### Additifs

La composition de l'invention peut contenir tous les additifs ou actifs classiquement utilisés dans les produits de nettoyage. On peut citer par exemple les conservateurs, notamment des conservateurs cationiques tels que la biguanide ; les séquestrants (EDTA) ; les antioxydant ; les parfums ; les matières colorantes telles que les colorants solubles, les pigments, les nacres ; les charges minérales ou organiques, apportant de la viscosité, matifiantes, blanchissantes ou exfoliantes ; les filtres solaires ; les actifs cosmétiques ou dermatologiques, hydrophiles ou lipophiles, tels que les vitamines hydrosolubles ou liposolubles, les antiseptiques, les antiséborrhéïques, les antimicrobiens tels que le peroxyde de benzoyle, l'acide salicylique, le triclosan, l'acide azélaïque, la niacinamide (vit. PP), et aussi les azurants optiques ; les polymères non ioniques, anioniques, cationiques et/ou amphotères ; les corps gras incompatibles avec le milieu aqueux, comme les huiles ou les cires ; les agents régulateurs de viscosité et agents épaississants ; des exfoliants ; des dispersions d'huile ou de corps gras ;ou d'autres agents ayant pour effet d'améliorer les propriétés cosmétiques de cheveux ou de la peau. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention et qu'ils ne la déstabilisent pas.

Comme charges, on peut citer notamment la silice, l'amidon, le nylon.

Comme exfoliants, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Ces particules peuvent être présentes en une quantité allant par exemple de 0,5 à 40 % en poids, de préférence de 1 à 20 % en poids et mieux de 1 à 10 % en poids par rapport au poids total de la composition. Quand la composition contient des particules exfoliantes, elle peut constituer notamment une composition de gommage de la peau du visage ou du corps.

Selon un mode préféré de réalisation de l'invention, la composition contient au moins un polymère cationique.

On peut citer par exemple comme polymères cationiques utilisables dans la composition de l'invention, les polymères comportant au moins un groupement amine quaternaire et éventuellement des groupements amine primaire, secondaire, tertiaire et/ou quaternaire faisant partie de la chaîne polymère ou directement reliés à celle-ci. Ces polymères ont généralement un poids moléculaire allant de 500 à environ 5.000.000 et de préférence de 1000 à 3.000.000.

Parmi ces polymères, on peut citer plus particulièrement les polymères cationiques suivants :
(1) les homopolymères ou copolymères dérivés d'esters ou d'amides acryliques ou méthacryliques et comportant au moins un groupe ammonium quaternaire, et en particulier un des motifs de formules suivantes : 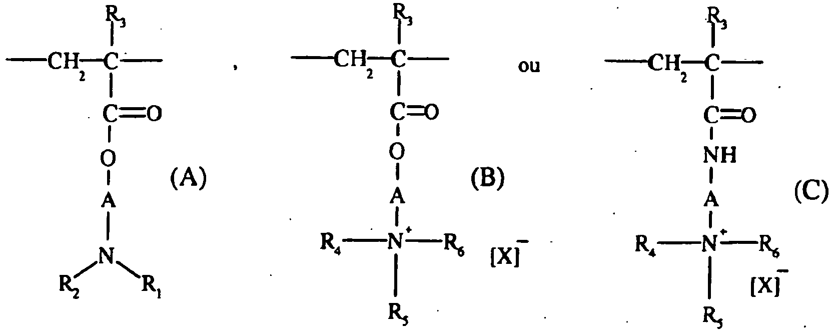 dans lesquelles :
   R3 désigne un atome d'hydrogène ou un radical CH3 ;
   A est un groupe alkyle linéaire ou ramifié de 1 à 6 atomes de carbone ou un groupe hydroxyalkyle comportant de 1 à 4 atomes de carbone ;
   R4, R5, R6, identiques ou différents, représentent un groupe allyle ayant de 1 à 18 atomes de carbone ou un radical benzyle ;
   R1 et R2 représentent un atome d'hydrogène ou un groupe alkyle ayant de 1 à 6 atomes de carbone ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer par exemple :
   - les copolymères d'acrylamide et de diméthylaminoéthyl méthacrylate quaternisé au sulfate de diméthyle ou avec un halogénure de diméthyle, tels que le produit commercialisé sous la dénomination HERCOFLOC par la société HERCULES ;
   - les copolymères d'acrylamide et de chlorure de méthacryloyloxyéthyltriméthylammonium, comme le POLYQUATERNIUM 5 (nom INCI) et par exemple le produit commercialisé sous la dénomination MERQUAT 5 par la société NALCO ; et comme le POLYQUATERNIUM 15 (nom INCI) et par exemple le produit commercialisé sous la dénomination ROHAGIT KF 720 F par la société ROHM;
   - le copolymère d'acrylamide et de méthosulfate de méthacryloyloxyéthyltriméthylammonium commercialisé sous la dénomination RETEN par la société HERCULES ;
   - les copolymères vinylpyrrolidone / acrylate ou méthacrylate de dialkylaminoalkyle quatemisés ou non, comme le POLYQUATERNIUM 11 (nom INCI) et par exemple les produits commercialisés sous les dénominations GAFQUAT 755, GAFQUAT 755N et GAFQUAT 734 par la société ISP ;
   - les terpolymères méthacrylate de diméthylaminoéthyie / vinylcaprolactame / vinylpyrrolidone tel que le produit vendu sous la dénomination GAFFIX VC 713 par la société ISP ;
   - le copolymère vinylpyrrolidone / méthacrylamide de diméthylaminopropyle quaternisé comme le POLYQUATERNIUM 28 (nom INCI) et par exemple le produit commercialisé sous la dénomination GAFQUAT HS-100 par la société ISP ;
   - les copolymères à base de vinylpyrrolidone et vinylcaprolactame comme le POLYQUATERNIUM 46 (nom INCI) et par exemple le produit commercialisé sous la dénomination LUVIQUAT HOLD par la société BASF ;
   - les terpolymères d'acide acrylique et de chlorure de (meth)acrylamidotriméthylammonium tel que le terpolymère acide acrylique / chlorure de methacrylamidopropyltrimethylammonium / acrylate de méthyle commercialisé par la société Nalco sous la dénomination Merquat 2001 (nom INCI: Polyquaternium 47).
(2) les homopolymères ou les copolymères de dimethyldiallylammonium de formule (2) décrite ci dessous : 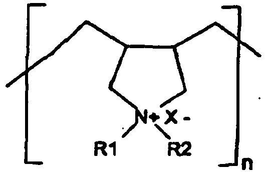 dans laquelle :
   R1 et R2, identiques ou différents, désignent un atome d'hydrogène, ou représentent un groupe alkyle ayant de 1 à 18 atomes de carbone
   X désigne un anion méthosulfate ou un halogénure tel que chlorure ou bromure.

   Les copolymères de la famille (2) contiennent en outre un ou plusieurs motifs dérivant de co-monomères pouvant être choisis dans la famille des acrylamides, méthacrylamides, diacétones acrylamides, acrylamides et méthacrylamides substitués sur l'azote par des radicaux alkyles inférieurs (C1-C6), des acides acryliques ou méthacryliques ou leurs esters, des vinyllactames tels que la vinylpyrrolidone ou le vinylcaprolactame, des esters vinyliques.
   Comme polymères de la famille (2), on peut citer par exemple les polymères suivants :
   - les polymères de chlorure de dimethyldiallyl ammonium, comme le POLYQUATERNIUM 6 (nom INCI) et par exemple les produits commercialisés sous les dénominations SALCARE SC 30 par la société CIBA, et MERQUAT 100 par la société NALCO ;
   - les copolymères d'acrylamide et de chlorure de diméthyldiallyl ammonium, comme le POLYQUATERNIUM 7 (nom INCI) et par exemple les produits commercialisés sous les dénominations MERQUAT S, MERQUAT 2200 et MERQUAT 550 par la société NALCO, SALCARE SC 10 par la société CIBA.
(3) les polysaccharides quaternisés tels que les gommes de guar contenant des groupements cationiques trialkylammonium, comme les produits commercialisés notamment sous les dénominations commerciales de JAGUAR C13 S, JAGUAR C 15, JAGUAR C 17, JAGUAR C 162, JAGUAR C 2000, JAGUAR EXCEL par la société MEYHALL ; ou tels que les dérivés de cellulose quaternisés, comme le polymère d'hydroxyethylcellulose contenant des groupes cationiques trialhylammonium, comme le POLYQUATERNIUM 10 (nom INCI) et par exemple le produit commercialisé sous la dénomination UCARE POLYMER JR-400 par la société AMERCHOL.
(4) les copolymères quaternaires de vinylpyrrolidone et de vinylimidazole ; comme le POLYQUATERNIUM 16 (nom INCI) et par exemple les produits commercialisés sous les dénominations LUVIQUAT FC905, LUVIQUAT FC370, LUVIQUAT HM552 et LUVIQUAT FC550 par la société BASF et le POLYQUATERNIUM 44 (nom INCI) et par exemple le produit commercialisé sous la dénomination LUVIQUAT CARE par la société BASF.
(5) les chitosanes ou leurs sels tels que les acétate, lactate, glutamate, gluconate ou le pyrrolidone carboxylate de chitosane. Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(6) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl-triméthylammonium, ou de diméthyl-diallylammonium. Les produits commercialisés répondant à cette définition sont plus particulièrement les produits vendus sous la dénomination "CELQUAT L 200" et "CELQUAT H 100" par la Société National Starch. et les mélanges de ces polymères cationiques.

Les polymères particulièrement préférés sont les Polyquaternium 5, Polyquaternium 7, Polyquaternium 28, Polyquaternium 39, Polyquaternium 44 et Polyquaternium 47, et leurs mélanges. On préfère tout particulièrement le Polyquaternium 7.

La quantité de polymère cationique (en matière active) doit être telle qu'elle n'affecte pas les propriétés de la composition et notamment qu'elle n'entraîne pas d'instabilité. Cette quantité peut aller par exemple de 0,1 à 1 % en poids et de préférence de 0,2 à 0,5 % en poids par rapport au poids total de la composition.

Selon un mode particulier de réalisation de l'invention, quand la composition de l'invention contient un conservateur, il s'agit de préférence d'un conservateur cationique. Les conservateurs cationiques ont l'avantage, outre leur propriété de protection contre les bactéries, d'améliorer la stabilité du gel. Comme conservateurs cationiques, on peut citer par exemple le chlorhydrate de poly-hexaméthylène biguanide (nom INCI : polyaminopropyl biguanide) ; les bromures d'alkyl-triméthylammonium, le radical alkyl comportant de 1 à 22 atomes de carbone, et plus particulièrement de 8 à 20 atomes de carbone comme par exemple le bromure de dodécyl triméthylammonium, le bromure d'hexadécyl triméthylammonium, le bromure de myristyl triméthylammonium (nom INCI: Mytrimonium bromide) qui peut se présenter en mélange avec d'autres bromures d'ammonium, par exemple dans le mélange de bromure de dodécyltriméthylammonium, de bromure de myristyl triméthyl ammonium et de bromure d'hexadécyl-triméthylammonium, vendu sous la dénomination Cetrimide par la société FEF CHEMICALS.

Les conservateurs sont présents dans la composition de l'invention en une quantité suffisante pour agir en tant que conservateur dans la composition. Ainsi, ils peuvent être présents en une quantité allant par exemple de 0,001 à 1 % du poids total de la composition, de préférence de 0,01 à 1 % du poids total de la composition et mieux de 0,05 à 0,5% du poids total de la composition.

Les compositions selon l'invention peuvent avoir une apparence allant du produit fluide à un gel. Elles sont stables et ont une très bonne rinçabilité. Elles peuvent constituer par exemple un produit de nettoyage et/ou de démaquillage de la peau y compris du cuir chevelu, et/ou des cheveux, un produit de gommage et/ou un produit exfoliant pour la peau (desquamation, peeling). Elles peuvent constituer plus particulièrement une composition de nettoyage de la peau.

L'invention a aussi pour objet l'utilisation cosmétique de la composition telle que définie ci-dessus, pour le nettoyage et/ou le démaquillage de la peau, et/ou des cheveux, et/ou pour le gommage de la peau.

Les compositions selon l'invention peuvent en particulier trouver des applications pour :
- le nettoyage profond
- le traitement des peaux grasses et / ou à problèmes
- Le nettoyage des peaux très fragiles
- les peelings mécaniques

Les compositions selon l'invention peuvent constituer notamment une composition pour le traitement des peaux mixtes à grasses, et on peut alors y ajouter un actif spécifique de traitement des peaux grasses, tels que, par exemple, les dérivés d'acide salicylique comme l'acide n-octanoyl salicylique, le gluconate de cuivre, la niacinamide (vitamine PP) et leurs mélanges.

Un autre objet de l'invention est un procédé cosmétique de traitement des peaux mixtes et grasses consistant à appliquer sur la peau, une composition telle que définie ci-dessus, et à rincer la peau.

Les compositions selon l'invention peuvent être utilisées de deux façons :
- la première utilisation consiste à étaler le gel dans les mains, à l'appliquer sur le visage ou sur le corps puis à le masser en présence d'eau pour développer la mousse directement sur le visage ou le corps.
- l'autre utilisation possible de ce type de produit consiste à développer la mousse dans les paumes des mains avant de l'appliquer sur le visage ou le corps.
Dans les deux cas, la mousse est ensuite rincée avec de l'eau ou essuyée avec une lingette, un gant, un coton.
La composition peut aussi être utilisée à sec sur les zones à traiter puis rincée à l'eau.
Les exemples qui suivent servent à illustrer l'invention sans toutefois présenter un caractère limitatif. Les quantités indiquées sont en % en poids sauf mention contraire.
Dans les tableaux ci-dessous, tous les pourcentages sont exprimés en poids de matière première (M.P.), la quantité de matière active (M.A.) étant précisée entre parenthèses.

### Exemples 1 à 4 selon l'invention

| Composition (% MP) | Exemple 1 | Exemple 2 | Exemple 3 | Exemple 4 |
|---|---|---|---|---|
| C12-C16 Alkyl Polyglucoside (1) | 17,16 (M.A. 8,58%) | 17,16 (M.A. 8,58%) | 17,16 (M.A. 8,58%) | 17,16 (M.A. 8,58%) |
| Lauryl dimethyl betaine (2) | 13 (M.A. 3,9 %) | 13 (M.A. 3,9 %) | 13 (M.A. 3,9 %) | 13 (M.A. 3,9 %) |
| Polyacrylate de sodium réticulé (3) | 2 | | | |
| Amidon modifié Acrylamide/Acrylate de sodium réticulé (4) | | 2 | | |
| Amidon/ gomme de guar/ sodium carboxymethyl cellulose (5) | | | 4 | |
| Amidon modifié Acrylamide/Acrylate de sodium réticulé (6) | | | | 2 |
| Copolymère de dimethyl diallyl ammonium/acrylamide (7) | | | | 2,87 (M.A. 0,249%) |
| Acide citrique | qsq pH7 | qsq pH7 | qsq pH7 | qsq pH7 |
| Eau | qsp 100% | qsp 100% | qsp 100% | qsp 100% |
| Aspect | Translucide | Translucide, givrée | Translucide | Translucide |
| Viscosité à 20°C (Rhéomètre Rheo RS150 0,1 s⁻¹) | 40 Pa.s | 270 Pa.s | 500 Pa.s | 300 Pa.s |
| (1) Plantacare 818 UP à 50% en M.A. | | | | |
| (2) Empigen BB / LS à 30% en M.A. | | | | |
| (3) Luquasorb 1010 (BASF) | | | | |
| (4) Waterlock C200 (Grain Processing) | | | | |
| (5) Lysorb 220 (Lysorb) | | | | |
| (6) Waterlock A100 (Grain Processing) | | | | |
| (7) Merquat S (Nalco) ou Polyquaternium-7 à 8,7% en M.A. | | | | |

Mode opératoire : le mélange de tensioactifs a été chauffé à 50°C puis on y a introduit le polymère superabsorbant. On a laissé gonfler 30 minutes et on a ajusté le pH avec l'acide citrique.

### Exemples 5 et 6 comparatifs

| Composition (% M.P.) | Exemple comparatif 5 | Exemple comparatif 6 |
|---|---|---|
| C12-C16 Alkyl Polyglucoside (1) | 17,16 (M.A. 8,58%) | 17,16 (M.A. 8,58%) |
| Lauryl dimethyl betaine (2) | 13 (M.A. 3,9 %) | 13 (M.A. 3,9 %) |
| Carbomer (8) | 2 | - |
| Amidon de mais modifié hydroxypropyl phosphate (9) | - | 2 |
| Acide citrique | Qs pH 7 | Qs pH 7 |
| Eau | qsp 100% | qsp 100% |
| Aspect | Gel opaque fluide | Solution translucide qui déphase en 24 heures |
| Viscosité à 20°C (Rhéomètre Rheo RS150 0,1 s⁻¹ | 72 Pa.s | Non mesurable |
| (1) Plantacare 818 UP à 50% en M.A. | | |
| (2) Empigen BB / LS à 30% en M.A. | | |
| (8) Carbopol 981 | | |
| (9)) Structure XL | | |

### Performances sensorielles :

Les performances sensorielles des compositions (qualités de mousse) ont été déterminées selon le protocole décrit ci-dessous.

Avant toute utilisation du produit, les mains sont lavées au savon de Marseille puis convenablement rincées et séchées. Puis le protocole suivi est le suivant :
1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- ajouter 2 ml d'eau et travailler le produit à nouveau pendant 10 secondes,
6- évaluer la qualité de la mousse selon les critères définis ci-dessous,
7- rincer les mains sous l'eau,
8- les essuyer.

Les critères de mousse sont notés sur une échelle de 0 à 10.
- étape 2 : évaluation du mélange à l'eau
- étapes 4-6 : évaluation de la qualité de mousse
- *L'homogénéité de la composition cisaillée entre les mains :* la note attribuée est d'autant plus élevée que l'homogénéité est bonne
- *Le démarrage de la mousse :* la note attribuée est d'autant plus élevée que le démarrage est bon
- *Le volume de mousse :* la note attribuée est d'autant plus élevée que le volume est grand.
- *La densité de la mousse :* la note attribuée est d'autant plus élevée que la densité est grande

Le panel d'évaluation est constitué de 5 experts entraînés. La moyenne des 5 notes permet de comparer les compositions selon chacun des critères.

| | Exemple 1 | Exemple 2 | Exemple 3 | Exemple 4 | Exemple comparatif 5 |
|---|---|---|---|---|---|
| Polymère | Luquasorb 1010 | Waterlock C200 | Lysorb 220 | Waterlock A100 | Carbopol 981 |
| Homogénéité | 10/10 | 10/10 | 10/10 | 10/10 | 7/10 |
| Démarrage de mousse | 5/10 | 5/10 | 5/10 | 7/10 | 0/10 |
| Volume de mousse | 6.5/10 | 7/10 | 6/10 | 10/10 | 2.3/10 |
| Densité de mousse | 7.5/10 | 8/10 | 6,5/10 | 8,5/10 | 2/10 |
| Taille de bulles | 3.5/10 | 3/10 | 3/10 | 4,5/10 | 3.2/10 |
| Résultat | + | + | + | + | - |

Le gel de l'exemple comparatif 5 à base de Carbopol conduit à des mousses moins homogènes, dont le démarrage n'est pas bon et le volume est très faible et de faible densité.

### Exemple 7 selon l'invention

| Composition (% MP) | Exemple 7 selon l'invention |
|---|---|
| C12-C16 Alkyl Polyglucoside (1) | 24,53 % |
| Polyacrylate de sodium réticulé (2) | 2 % |
| Polyaminopropyl biguanide (3) | 0,5 % |
| Acide citrique | qs pH 6,5 |
| Eau | qsp 100 % |
| Stabilité après 2 mois à toutes températures | stable |
| (1) Plantacare 818 UP 50% MA | |
| (2) Luquasorb 1010 (BASF) | |
| (3) Cosmocil CQ 20% MA | |

## Revendications

1. Composition de nettoyage pour application topique, se présentant sous forme d'un gel et contenant, dans un milieu aqueux physiologiquement acceptable, au moins 40% en poids d'eau par rapport au poids total de la composition, au moins un tensioactif moussant choisi parmi les tensioactifs non ioniques et les tensioactifs amphotères, et au moins un polymère superabsorbant se présentant sous forme de particules qui, une fois hydratées, gonflent en formant des billes molles ayant un diamètre moyen en nombre de 10 µm à 1000 µm **caractérisée en ce qu'**elle est exempte de tensioactif anionique.

2. Composition selon la revendication 1, **caractérisée en ce que** le polymère superabsorbant est choisi parmi les polyacrylates de sodium réticulés, les amidons greffés par un polymère acrylique, les amidons hydrolysés greffés par un polymère acrylique, les polymères à base d'amidon, de gomme et de dérivé cellulosique, et leurs mélanges.

3. Composition selon la revendication 1 ou 2, **caractérisée en ce que** la quantité de polymère(s) superabsorbant(s) va de 0,1 à 20 % en poids et de préférence de 0,5 à 20 % en poids par rapport au poids total de la composition.

4. Composition selon l'une quelconque des revendications précédentes, **caractérisée en ce que** les tensioactifs non ioniques sont choisis parmi les alkyl polyglucosides, les esters de maltose, les alcools gras polyglycérolés, les dérivés de glucamine, et leurs mélanges.

5. Composition selon l'une quelconque des revendications précédentes, **caractérisée en ce que** les tensioactifs amphotères sont choisis parmi les dérivés de bétoine, les alkylamphoacétates, les hydroxylsultaines, et leurs mélanges.

6. Composition selon l'une quelconque des revendications précédentes, **caractérisée en ce qu'**elle contient au moins un tensioactif non ionique et au moins un tensioactif amphotère.

7. Composition selon l'une quelconque des revendications précédentes, **caractérisée en ce que** la quantité totale de tensioactif(s) va de 3 à 20 % en poids par rapport au poids total de la composition.

8. Composition selon l'une quelconque des revendications précédentes, **caractérisée en ce qu'**elle contient en outre au moins un polymère cationique.

9. Composition selon la revendication précédente, **caractérisée en ce que** le polymère cationique est choisi parmi le Polyquaternium 5, le Polyquaternium 7, le Polyquaternium 28, le Polyquaternium 39, le Polyquaternium 44, le Polyquaternium 47, et leurs mélanges.

10. Composition selon la revendication 9 ou 10, **caractérisée en ce que** la quantité de polymère(s) cationique(s) va de 0,1 % en poids et de préférence de 0,2 à 5 % en poids par rapport au poids total de la composition.

11. Composition selon l'une quelconque des revendications précédentes, **caractérisée en ce qu'**elle constitue un produit de nettoyage et/ou de démaquillage de la peau, du cuir chevelu, des cheveux, et/ou un produit de gommage, ou un produit exfoliant pour la peau.

12. Utilisation cosmétique d'une composition selon l'une quelconque des revendications 1 à 10, pour le nettoyage et/ou le démaquillage de la peau, et/ou des cheveux, et/ou pour le gommage de la peau.

13. Procédé cosmétique de traitement des peaux mixtes et grasses consistant à appliquer sur la peau, une composition selon l'une quelconque des revendications 1 à 10, et à rincer la peau.

## Patentansprüche

1. Zusammensetzung für die Reinigung zur topischen Anwendung, die als Gel vorliegt und in einem physiologisch akzeptablen, wässrigen Medium mindestens 40 Gew.-% Wasser, bezogen auf das Gesamtgewicht der Zusammensetzung, mindestens einen schaumbildenden grenzflächenaktiven Stoff, der unter den nichtionischen grenzflächenaktiven Stoffen und den amphoteren grenzflächenaktiven Stoffen ausgewählt ist, und mindestens ein superabsorbierendes Polymer, das als Partikel vorliegt, die, sobald sie hydratisiert sind, unter Bildung von weichen Kugeln mit einem zahlenmittleren Durchmesser von 10 µm bis 1000 µm aufblähen, enthält, **dadurch gekennzeichnet, dass** kein anionischer grenzflächenaktiver Stoff enthalten ist

2. Zusammensetzung nach Anspruch 1, **dadurch gekennzeichnet, dass** das supcrabsorbierende Polymer unter den vernetzten Natriumpolyacrylaten, mit einem Acrylpolymer gepfropften Stärken, mit einem Acrylpolymer gepfropften, hydrolysierten Stärken, Polymeren auf Stärke-, Gummi- und Cellulosederivatbasis und deren Gemischen ausgewählt ist.

3. Zusammensetzung nach Anspruch 1 oder 2, **dadurch gekennzeichnet, dass** der Mengenanteil des oder der superabsorbierenden Polymere(s) im Bereich von 0,1 bis 20 Ges.-% und vorzugsweise 0,5 bis 20 Gew.-%, bezogen das der Zusammen setzung, liegt.

4. Zusammensetzung nach einem der vorhergehenden Ansprüche, **dadurch gekennzeichnet, dass** die nichtionischen grenzflächenaktiven Stoffe unter den Alkylpolyglucosiden, Estern von Maltose, mehrfach mit Glycerin veretherten Fettalkoholen, Glucaminderivaten und deren Gemische, ausgewählt sind.

5. Zusammensetzung nach einem der vorhergehenden Ansprüche, **dadurch gekennzeichnet, dass** die amphoteren grenzflächenaktiven Stoffe unter den Betainderivaten, Alkylamphoacetaten, Hydroxysultainen und deren Gemischen ausgewählt sind.

6. Zusammensetzung nach einem der vorhergehenden Ansprüche, **dadurch gekennzeichnet, dass** sie mindestens einen nichtionischen grenzflächenaktiven Stoff und mindestens einen amphoteren grenzflächenaktiven Stoff enthält.

7. Zusammensetzung nach einem der vorhergehenden Ansprüche, **dadurch gekennzeichnet, dass** die Gesamtmenge des (der) grenzflächenaktiven Stoffe(s) im Bereich von 3 bis 20 Gew.-%, bezogen auf das Gesamtgewicht der Zusammensetzung, liegt.

8. Zusammensetzung nach einem der vorhergehenden Ansprüche, **dadurch gekennzeichnet, dass** sie ferner mindestens ein kationisches Polymer enthält.

9. Zusammensetzung nach dem vorhergehenden Anspruch, **dadurch gekennzeichnet, dass** das kationische Polymer unter Polyquaternium 5, Polyquaternium 7, Polyquaternium 28, Polyquaternium 39, Polyquaternium 44, Polyquaternium 47 und deren Gemischen ausgewählt ist.

10. Zusammensetzung nach Anspruch 9 oder 10, **dadurch gekennzeichnet, dass** der Mengenanteil des (der) kationischen Polymere(s) im Bereich von 0,1 und vorzugsweise 0,2 bis 5 Gew.-%, bezogen auf das Gesamtgewicht der Zusammensetzung, liegt.

11. Zusammensetzung nach einem, der vorhergehenden Ansprüche, **dadurch gekennzeichnet, dass** es sich um eine Produkt für die Reinigung und/oder zum Abschminken der Haut, der Kopfhaut der Haare und/oder ein abradierendes Produkt und/oder ein Peeling-Produkt für die Haut handelt.

12. Kosmetische Verwendung einer Zusammensetzung nach einem der Ansprüche 1 bis 10 für die Reinigung und/oder zum Abschminken der Haut und/oder der Kopfhaut und/oder ein abradierendes Produkt für die Haut.

13. Kosmetisches Verfahren zur Behandlung vom Mischhaut und fettiger Haut, das darin besteht, eine Zusammensetzung nach einem der Ansprüche 1, bis 10 auf die Haut aufzubringen und die Haut zu spülen.

## Claims

1. Cleansing composition for topical application, in the form of a gel and containing, in a physiologically acceptable aqueous medium, at least 40% by weight of water relative to the total weight of the composition, at least one foaming surfactant chosen from nonionic surfactants and amphoteric surfactants, and at least one superabsorbent polymer, in the form of particles that swell, once they are hydrated, forming soft beads with a number average diameter of 10 µm to 1000 µm, **characterized in that** it is free of an anionic surfactant.

2. Composition according to Claim 1, **characterized in that** the superabsorbent polymer is chosen from crosslinked sodium polyacrylates, starches grafted with an acrylic polymer, hydrolysed starches grafted with an acrylic polymer, polymers based on starch, on gum and on a cellulose derivative, and mixtures thereof.

3. Composition according to Claim 1 or 2, **characterized in that** the amount of superabsorbent polymer(s) ranges from 0.1% to 20% by weight, and preferably from 0.5% to 20% by weight, relative to the total weight of the composition.

4. Composition according to any one of the preceding claims, **characterized in that** the nonionic surfactants are chosen from alkyl polylglucosides, maltose esters, polyglycerolated fatty alcohols, glucamine derivatives, and mixtures thereof.

5. Composition according to any one of the preceding claims, **characterized in that** the amphoteric surfactants are chosen from betaine derivatives, alkyl amphoacetates, hydroxysultaines, and mixtures thereof.

6. Composition according to any one of the preceding claims, **characterized in that** it contains at least one nonionic surfactant and at least one amphoteric surfactant.

7. Composition according to any one of the preceding claims, **characterized in that** the total amount of surfactant(s) ranges from 3% to 20% by weight relative to the total weight of the composition.

8. Composition according to any one of the preceding claims, **characterized in that** it also contains at least one cationic polymer.

9. Composition according to the preceding claim, **characterized in that** the cationic polymer is chosen from polyquaternium 5, polyquaternium 7, polyquaternium 28, polyquaternium 39, polyquaternium 44, polyquaternium 47, and mixtures thereof.

10. Composition according to Claim 9 or 10, **characterized in that** the amount of cationic polymer(s) ranges from 0.1% by weight, and preferably from 0.2% to 5% by weight, relative to the total weight of the composition.

11. Composition according to any one of the preceding claims, **characterized in that** it constitutes a cleansing and/or makeup-removing product for the skin, the scalp and/or the hair, and/or a scrubbing product or an exfoliant product for the skin.

12. Cosmetic use of a composition according to any one of Claims 1 to 10, for cleansing and/or removing makeup from the skin and/or the hair, and/or for scrubbing the skin.

13. Cosmetic process for the treatment of combination and greasy skin, consisting in applying, to the skin, a composition according to any one of Claims 1 to 10, and in rinsing the skin.
